# EUROPEAN PATENT SPECIFICATION

(11) **EP 3 530 645 B1**
(45) Date of publication and mention of the grant of the patent: **06.12.2023**
(21) Application number: 17862001.9
(22) Date of filing: 16.10.2017
(51) Int. Cl.: C07C 221/00, C07C 225/14, C07D 231/12

(54) **METHOD FOR PRODUCING NITROGEN-CONTAINING COMPOUND**
VERFAHREN ZUR HERSTELLUNG EINER STICKSTOFFHALTIGEN VERBINDUNG
PROCÉDÉ DE PRODUCTION D'UN COMPOSÉ CONTENANT DE L'AZOTE

(30) Priority: 19.10.2016 JP 2016205020; 27.03.2017 JP 2017061391
(43) Date of publication of application: 28.08.2019
(73) Proprietor: AGC INC., Chiyoda-ku, Tokyo 1008405 (JP)
(72) Inventor: YAMAZAKI, Yusuke, Tokyo 100-8405 (JP); SAWAGUCHI, Masanori, Tokyo 100-8405 (JP); ISHIBASHI, Yuichiro, Tokyo 100-8405 (JP)
(74) Representative: Müller-Boré & Partner Patentanwälte PartG mbB
(86) International application number: PCT/JP2017/037350
(87) International publication number: WO 2018/074411

(56) References cited:
- WO-A1-2016/152886
- WO-A1-2016/152886
- CN-A- 101 781 222
- JP-A- 2015 511 944
- US-A1- 2003 171 622
- J.T. GUPTON, ET AL.: "[[[(Dimethylamino)methylene]amino]methyle ne]dimethylammonium chloride" In: L. Paquette (ed.): "e-EROS Encyclopedia of Reagents for Organic Synthesis", 15 April 2001 (2001-04-15), John Wiley & Sons, New York, NY, US, XP009519886, ISBN: 9780471936237 pages 1-3, DOI: 10.1002/047084289X.rd304m, * scheme 5; page 1, left-hand column *
- J.T. GUPTON, ET AL.: "Reactions of [3-(dimethylamino)-2-azaprop-2-en-1-yliden e]dimethylammonium chloride with methyl ketones, primary amines, and unsubstituted amides", JOURNAL OF ORGANIC CHEMISTRY, vol. 45, no. 22, October 1980 (1980-10), pages 4522-4524, XP055685181, American Chemical Society, Washington, DC, US ISSN: 0022-3263, DOI: 10.1021/jo01310a058
- SCHUPPE, A. W. ET AL.: 'Scalable synthesis of enaminones utilizing Gold's reagents' TETRAHEDRON vol. 73, no. 26, June 2017, pages 3643 - 3651, XP055478128

## Description

The present invention relates to a production method of a nitrogen-containing compound useful as an optical material and a starting material for pharmaceuticals/agrochemicals.

### [Background Art]

A nitrogen-containing compound having a - C(O)CH=CHN<structure is useful as an optical material or a starting material for pharmaceuticals/agrochemicals. For example, CH₃C(O)CH=CHN(CH₃)₂ is useful as a starting material of 3-difluoromethyl-1-methyl-1H-pyrazole-4-carboxylic acid used for pyrazolylcarboxanilide antimicrobial agents (see patent document 1). Patent document 2 describes the preparation of [α]-dicarbonyl compounds in particular for the preparation of 2,3-pentanedione.

As a production method of CH₃C(O)CH=CHN(CH₃)₂, non-patent document 1 describes a method including reacting [N(CH₃)₂CH=NCHN(CH₃)₂]⁺·Cl⁻ with a methanol solution of sodium methoxide and reacting the obtained N(CH₃)₂CH=NCH(OCH₃) (N(CH₃)₂) with CH₃C(O)CH₃. Non-patent document 2 describes the synthesis of [[[(dimethylamino)methylene]amino]methylene]dimethylammonium chloride and its reactivity with nucleophiles. Non-patent document 3 describes reactions of [3-(dimethylamino)-2-azaprop-2-en-1-ylidene]dimethylammonium chloride with methyl ketones, primary amines, and unsubstituted amides.

### [Document List]

### [Patent document]

patent document 1: WO2016/152886
patent document 2: US 2003/0171622 A1

### [non-patent document]

non-patent document 1: Bull. Soc. Chim. Belg, 1994, page 697 - page 703
non-patent document 2: J. T. Gupton et al., in L. Paquette (ed.) e-EROS Encyclopedia of Reagents for Organic Synthesis, DOI: 10.1002/047084289X.rd304m.
non-patent document 3: J. T. Gupton et al., J. Org. Chem 1980, 45, 4522-4524.

With the method and conditions described in non-patent document 1, however, CH₃C(O)CH=CHN(CH₃)₂ is obtained only in a low yield.

The problem of the present invention is to provide a production method of a nitrogen-containing compound, which can produce a nitrogen-containing compound useful as an optical material or a starting material for pharmaceuticals/agrochemicals industrially efficiently from an easily obtainable, economical compound.

The present inventors have conducted intensive studies regarding a method for producing a nitrogen-containing compound efficiently and economically and found that a nitrogen-containing compound can be produced efficiently and economically by reacting a compound represented by the below-mentioned formula (4), alkylketone (a compound represented by the below-mentioned formula (2)), N,N-dialkylformamide (a compound represented by the below-mentioned formula (3)), all of which are easily obtainable and economical compounds, under particular conditions. They have further found that a highly pure nitrogen-containing compound can be produced efficiently and economically by purifying the reaction product under particular conditions.

That is, the present inventors have found an efficient and economical production method of a nitrogen-containing compound by using easily obtainable and economical compounds. Accordingly, the present invention is as defined in claims 1 to 10.

According to the production method of the present invention, a nitrogen-containing compound useful as an optical material or a starting material for pharmaceuticals/agrochemicals can be produced industrially efficiently from an easily obtainable, economical compound.

In the present specification, a compound represented by the formula (X) is indicated as compound (x). In the present specification, a numerical range indicated using "-" refers to a range including the numerical values indicated before and after "-" respectively as the minimum value and the maximum value.

The embodiment of the present invention is described in detail below.

The present invention provides a method for producing the following compound (1) by reacting the following compound (4) with 8- to 40-fold moles of the following compound (3) to give a reaction mixture of the following compound (4) and the following compound (3), and reacting the reaction mixture with the following compound (2) by using a basic compound. In the present specification, compound (1) is a "nitrogen-containing compound" which is also referred to as ketoenamine. 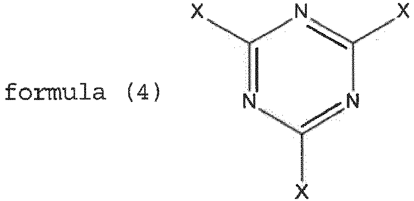
the

   formula (3) NR¹R²C(O)H
the

   formula (2) R³C(O)CH₃
the

   formula (1) R³C(O)CH=CHNR¹R²
X is a halogen atom, preferably a chlorine atom, a bromine atom or an iodine atom, and more preferably a chlorine atom.

Three X's may be the same or different, are preferably the same, and more preferably the same and chlorine atoms. That is, compound (4) is preferably cyanuric chloride.

R¹ and R² are each independently an alkyl group having 1 - 6 carbon atoms.

The alkyl group having 1 - 6 carbon atoms for R¹ or R² may be linear or branched, and is preferably an alkyl group having 1 - 3 carbon atoms, more preferably a methyl group.

R¹ and R² may be the same or different, are preferably the same, and more preferably the same and methyl groups. That is, compound (3) is preferably dimethylformamide.

R³ is a methyl group. That is, compound (2) is acetone.

In compound (1), the steric configuration of R³C(O)- and NR¹R²-, which are bonded to -CH=CH-, may be cis or trans.

The reaction mixture of compound (4) and compound (3) in the present invention (hereinafter to be referred to as "reaction mixture") is explained in detail below.

The reaction mixture is formed by the reaction of compound (4) and compound (3) and accompanying decarboxylation, and is considered to contain a salt represented by the formula [NR¹R²CH=NCH=NR¹R²]⁺·X⁻ (wherein R¹, R² and X⁻ are as defined above, hereinafter the same). The stoichiometry in the salt formation reaction is 6-fold moles of compound (3) relative to compound (4).

In the present invention, 8- to 40-fold moles of compound (3) relative to compound (4), namely, compound (3) in an amount exceeding stoichiometry is used to prepare the reaction mixture. Therefore, the reaction mixture in the present invention contains compound (3) exceeding the stoichiometry. The present inventors have found that the amount of compound (3) exceeding the stoichiometry as a solvent promotes dissolution (preferably uniform dissolution) of the above-mentioned salt, and that compound (1) as a nitrogen compound that can also be called ketoenamine is efficiently obtained in the reaction of the reaction mixture in such a solution state and compound (2).

The reaction mixture in the present invention is obtained by reacting 8- to 40-fold moles, more preferably 10- to 30-fold moles, of compound (3) relative to compound (4). Within this range, compound (1) tends to be obtained particularly efficiently in a high yield.

From the aspect of adjusting the solution state of the reaction mixture, it is preferable to obtain the reaction mixture of compound (4) and compound (3) in the presence of ether. Ether is used as a solvent. As the ether, ether as an aprotic and saturated compound is preferable. Ether may be cyclic ether or chain ether. Specific examples of ether include diethyl ether, tert-butyl methyl ether, dioxane, tetrahydrofuran, and cyclopentyl methyl ether. When a reaction mixture is prepared in the presence of ether, the amount of ether to be used is preferably not less than 1-fold volume based on the volume of compound (4). The upper limit thereof is not particularly limited, and not more than 10-fold volume is preferable and, from the aspect of volume efficiency, not more than 2-fold volume is more preferable.

The temperature during preparation of the reaction mixture is preferably 0 - 200°C, more preferably 30 - 100°C. The pressure during preparation of the reaction mixture is not particularly limited, and the reaction is generally performed under atmospheric pressure.

The reaction of the reaction mixture and compound (2) using a basic compound in the present invention is explained in detail below.

In the reaction, the amount of compound (2) to be used is preferably not less than 3-fold moles, more preferably more than 3-fold moles, based on the amount of substance of compound (4) used for preparing the reaction mixture. The upper limit of the amount of use is not particularly limited, and not more than 100-fold moles is preferable, not more than 20-fold moles is more preferable. Within this range, compound (1) tends to be obtained efficiently in a high yield.

The basic compound is not particularly limited as long as it activates R¹ and R² of compound (3), and alkali metal alkoxide, tertiary amine or alkali metal hydride is preferable, and alkali metal alkoxide is more preferable.

In the alkali metal alkoxide, alkali metal is preferably sodium or potassium, and alkoxide is preferably methoxide, ethoxide or isopropoxide. The alkali metal alkoxide is preferably sodium methoxide. Also, the use form of the alkali metal alkoxide is not particularly limited, and alkali metal alkoxide may be used as it is (solid alkali metal alkoxide alone) or an alcohol solution of alkali metal alkoxide, and alkali metal alkoxide is preferably used as it is.

Examples of the tertiary amine include trialkyl amine having an alkyl group having 1 - 4 carbon atoms, imidazole, pyridine, 2,6-lutidine, s-collidine, N-methylpyrrolidine, and N-methylpiperidine. Trialkyl amine having an alkyl group having 1 - 4 carbon atoms is preferable, triethylamine, tributylamine or ethyldiisopropylamine is more preferable, and triethylamine is further preferable.

The alkali metal hydride is preferably LiAlH₄, NaBH₄, NaH or LiN(CH(CH₃)₂)₂.

The amount of the basic compound to be used in the reaction is preferably not less than 3-fold moles, more preferably more than 3-fold moles, based on compound (4) used for preparing the reaction mixture. The upper limit of the amount of use is not particularly limited, and not more than 6-fold moles is preferable. Within this range, compound (1) is obtained efficiently in a high yield.

In the reaction, while an amount exceeding the stoichiometry of compound (3) used for preparation of the reaction mixture is present as a solvent as mentioned above, the reaction may be performed in the presence of a still other organic solvent. The other organic solvent is not particularly limited, and is appropriately determined according to the kind of the basic compound. When the basic compound is alkali metal alkoxide, the above-mentioned ether or alcohol can be mentioned. Specific examples of alcohol include methanol, ethanol, n-propanol, iso-propanol, n-butanol, and iso-butanol. When the basic compound is tertiary amine or alkali metal hydride, aromatic hydrocarbon or saturated aliphatic hydrocarbon is preferable. Specific examples of aromatic hydrocarbon include toluene and xylene. Specific examples of saturated aliphatic hydrocarbon include heptane and hexane.

The total amount of the solvent used in the reaction is preferably not less than 1-fold volume based on compound (4) used for preparing the reaction mixture. The upper limit is not particularly limited, and not more than 20-fold volume is preferable. Within this range, compound (1) tends to be obtained efficiently in a high yield. The total amount of the above-mentioned solvent to be used is the total amount including compound (3) in an amount exceeding the stoichiometry and other organic solvent.

The temperature of the reaction is preferably -78 to +200°C, more preferably -60 to +100°C, further preferably not less than -30°C and less than 60°C, particularly preferably -30 to +40°C. The pressure during preparation of the reaction mixture is not particularly limited, and the reaction is generally performed under atmospheric pressure. The reaction time is not particularly limited, and the reaction may be ceased when the reaction end-point is reached.

As a specific embodiment of the production method of the present invention, an embodiment in which 8- to 40-fold moles of compound (3) is reacted with compound (4) to give a reaction mixture of compound (4) and compound (3) and, without essentially removing compound (3) contained in the reaction mixture, the reaction mixture is reacted with compound (2) by using a basic compound can be mentioned. In the embodiment, the basic compound is alkali metal alkoxide of alcohol represented by the formula R^{A}-OH (wherein R^{A} is a methyl group, an ethyl group or an isopropyl group), and it is preferable to form amino acetal represented by the formula NR1R²CH=NCH(OR^{A}) (N(R¹R²)) from the reaction mixture.

In the reaction of the reaction mixture and compound (2), an embodiment in which a basic compound is added to the reaction mixture, compound (2) is added and the reaction mixture is reacted with compound (2) is preferable. That is, an embodiment in which the reaction mixture is mixed with a basic compound to give a mixture, the mixture is mixed with compound (2), and the reaction mixture is reacted with compound (2) is preferable.

In the embodiment, the basic compound is preferably the above-mentioned alkali metal alkoxide.

As mentioned above, the reaction of the reaction mixture and compound (2) can be performed by selecting various preferable conditions.

When the basic compound is alkali metal alkoxide, the amount of compound (2) to be used is preferably more than 3-fold moles and not more than 20-fold moles (preferably 4- to 12-fold moles), or more than 12-fold moles (preferably 16- to 100-fold moles, more preferably 24- to 72-fold moles), based on the amount of substance of compound (4) used for preparing the reaction mixture.

When the amount of compound (2) to be used is within the aforementioned range, compound (2) is in excess relative to the salt formed by the reaction of the above-mentioned compound (4) and compound (3), which is contained in the reaction mixture, the reaction activity is enhanced since the above-mentioned salt, compound (2) and alkoxide are highly compatible, and compound (1) is easily obtained efficiently in a high yield. Within this range, the reaction temperature in the reaction of the reaction mixture and compound (2) is preferably more than 40°C, more preferably not less than 60°C. The above-mentioned reaction temperature is preferably not more than 200°C, more preferably not more than 100°C.

When the amount of compound (2) to be used is within the below range, compound (2) contained in excess as a polar solvent promotes compatibility of the above-mentioned salt and alkali metal alkoxide and enhances reaction activity, as a result of which compound (1) tends to be easily obtained efficiently in a high yield. The reaction temperature in the reaction of the reaction mixture and compound (2) is preferably not more than the boiling point of compound (2), more preferably not less than -30°C and less than 60°C, further preferably -30 to +40°C.

As mentioned above, as alkali metal alkoxide, solid alkali metal alkoxide itself is preferably used. While the reason therefor is not necessarily clear, when solid alkali metal alkoxide itself is used, decomposition of the above-mentioned aminoacetal formed by the reaction mixture and alkali metal alkoxide, which is induced by the presence of alcohol, is suppressed as compared to the use of an alcohol solution of alkali metal alkoxide.

In the reaction of the reaction mixture and compound (2) in the present invention, a compound represented by the formula NR¹R²CH=NH (hereinafter to be referred to as "aldimine" wherein R¹ and R² mean the same as above) is produced simultaneously with compound (1), and therefore, compound (1) and aldimine are coexistent in the reaction product obtained by the production method of the present invention. Aldimine in the present invention is a compound having a low boiling point as compared to compound (1).

Aldimine reacts with water by-produced in the present production method and may be converted to compound (3).

It is preferable to produce highly pure compound (1) from the compound (1)-containing reaction product obtained by the production method of the present invention by reducing or removing, by an evaporation operation, a compound having a low boiling point (e.g., aldimine, solvent and the like) as compared to compound (1) and contained in the reaction product (hereinafter to be referred to as "low boiling point compound").

The temperature of the evaporation operation is preferably less than 70°C, more preferably less than 60°C, further preferably not more than 30°C. Within the range of the upper limit of the temperature, compound (1) can be made to have high purity efficiently in a high yield. While the reason therefor is not necessarily clear, it is considered to be because by-production of a side reaction product of aldimine coexisting with the reaction product and compound (1) is suppressed within the above-mentioned upper limit range of the temperature. Specific examples of the above-mentioned side reaction product include compounds represented by the following formula (py) and the following formula (ap).

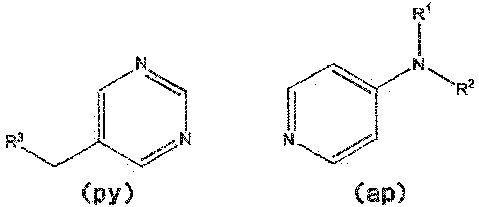

The lower limit of the temperature of the evaporation operation is not particularly limited. It is generally not less than 0°C, and compound (1) can be easily made to have high purity efficiently in a high yield within the range of 0 - 30°C.

The pressure in the evaporation operation is not particularly limited. From the aspect of efficient progress of evaporation, it is generally performed under reduced pressure condition.

Furthermore, it is preferable to further purify a crudely purified product in which a low boiling point compound is reduced or removed, which is obtained by the evaporation operation of the reaction product, by distillation to obtain purified highly pure compound (1) as a fraction thereof.

The temperature of the distillation operation is not particularly limited, and not more than 180°C is preferable, and not more than 140°C is more preferable. While the lower limit thereof is not particularly limited, it is generally not less than 40°C. The pressure of the distillation operation is not particularly limited and, from the aspect of efficiency, it is generally performed under reduced pressure conditions.

Using compound (1) obtained by the production method of the present invention, a compound useful as an optical material or a pharmaceutical/agrochemical intermediate can be produced.

Specifically, the following compound (8) can be produced by obtaining compound (1) by the production method of the present invention, reacting the compound (1) with the following compound (5) to give the following compound (6) and reacting the compound (6) with the following compound (7):
the

   formula (5) R^{F}C(O)Z
the

   formula (7) R⁴NHNH₂

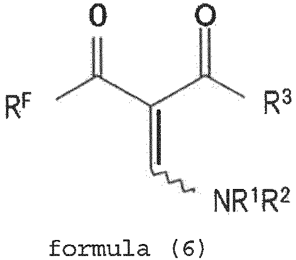

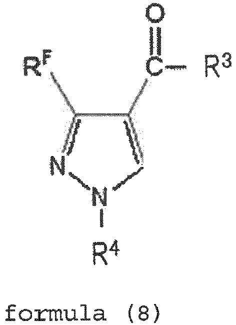

Z is a fluorine atom or a chlorine atom, and a fluorine atom is preferable.

R^{F} is a haloalkyl group having 1 - 3 carbon atoms, preferably a difluoromethyl group, a chlorodifluoromethyl group, a dichloromethyl group, a trifluoromethyl group or a trichloromethyl group, more preferably a difluoromethyl group.

R¹ - R³ mean the same as above, and a preferable range thereof is the same.

R⁴ is an alkyl group having 1 - 3 carbon atoms, preferably an n-propyl group, an iso-propyl group, an ethyl group or a methyl group, more preferably a methyl group.

By oxidizing compound (8), highly pure 3-haloalkyl-1-alkyl-1H-pyrazole-4-carboxylic acid (particularly, 3-difluoromethyl-1-methyl-1H-pyrazole-4-carboxylic acid) useful as a starting material for pharmaceuticals/agrochemicals can be easily produced.

As described above, according to the production method of the present invention, a nitrogen-containing compound can be produced efficiently in a high yield from an easily obtainable economical compound as a starting material. That is, the production method of the present invention is an economical production method of a nitrogen-containing compound useful as an optical material or a starting material for pharmaceuticals/agrochemicals.

### [Examples]

While the present invention is described in the following by way of Examples, the present invention is not limited to these Examples.

### [Example 1]

Under an air atmosphere, cyanuric chloride (38.4 g) and dimethylformamide (198 g) are placed in a flask, and the content of the flask is stirred for 2 hr at a flask inside temperature of 60°C to give a reaction mixture. Then, the inside temperature of the flask is maintained at 40°C, a solution of sodium methoxide in methanol which is obtained by mixing sodium methoxide (33.8 g) and methanol (276 g) is added to the flask while stirring the content of the flask, and the mixture is maintained as it is for 1 hr. Then, the inside temperature of the flask is maintained at 25°C, acetone (36.3 g) is added to the flask while stirring the content of the flask, and the mixture is reacted as it is for 18 hr.

The content of the flask is analyzed. As a result, it is confirmed that CH₃C(O)CH=CHN(CH₃)₂ (hereinafter to be also indicated as compound (1¹)) is produced in a yield of 68% based on cyanuric chloride. The content of the flask is filtered and the filtrate is recovered to give a reaction mixture containing CH₃C(O)CH=CHN(CH₃)₂.

### [Examples 2 and 3 and Reference Examples 4 and 5]

In the same manner as in Example 1 except that the charged amount of each component and the presence or absence of the use of other organic solvent are changed, compound (1^{l}) was synthesized. The results are collectively shown in Table 1. As other organic solvent in Table 1, tetrahydrofuran is used in Examples 3 and 4 and isopropyl alcohol is used in Example 5.

**[Table 1]**

| Example | reaction temperature [°C] | DMF amount [eq] | other organic solvent | | yield [%] |
|---|---|---|---|---|---|
| | | | [presence or absence (kind)] | [volume ratio] | |
| Example 1 | 25 | 12 | absent | - | 68 |
| Example 2 | 60 | 12 | absent | - | 57 |
| Example 3 | 60 | 8 | present (ether) | 1.3 | 59 |
| Example 4^{◆} | 60 | 6.0 | present (ether) | 1.3 | 45 |
| Example 5^{◆} | 60 | 6.0 | present (alcohol) | 1.3 | 3 |

| | | | | | |
|---|---|---|---|---|---|
| * DMF amount [eq] is molar amount based on cyanuric chloride. * Volume ratio of other organic solvent is volume ratio based on volume of cyanuric chloride. * Yield [%] is yield in moles of compound (1¹) based on cyanuric chloride. ◆ Reference Example | | | | | |

### [Example 6]

A low boiling point compound in the reaction mixture is removed from the reaction mixture obtained in Example 1 by an operation of evaporation under reduced pressure under the conditions of temperature 20 - 30°C, pressure 10 - 30 Pa to give a crudely purified product. The crudely purified product is further evaporated under reduced pressure to give, as a fraction, compound (1¹) having a purity of more than 99% in a distillation yield of 93%, and a yield of 63% based on cyanuric chloride.

### [Example 7]

A low boiling point compound in the reaction mixture is removed from the reaction mixture obtained in Example 1 by an operation of evaporation under reduced pressure under the conditions of temperature 70 - 90°C, pressure 12000 - 20000 Pa to give a crudely purified product. The crudely purified product is further evaporated under reduced pressure to give, as a fraction, compound (1¹) having a purity of more than 99% in a distillation yield of 74%, and a yield of 50% based on cyanuric chloride.

As is clear from a comparison of Examples 1 - 3 and Reference Examples 4 - 5, it is known that compound (1) is obtained in a high yield when a reaction mixture obtained by reacting 8- to 40-fold moles of dimethylformamide which is one embodiment of compound (3) relative to cyanuric chloride which is one embodiment of compound (4) is used. In addition, as is clear from a comparison of Example 6 and Example 7, it is known that compound (1) is obtained in a high yield when the obtained reaction product is subjected to an evaporation operation at a low temperature (20 - 30°C) as compared to when subjected to an evaporation operation at a high temperature (70 - 90°C).

### [Example 9]

Under an air atmosphere, cyanuric chloride (12.8 g) and dimethylformamide (198 g) are placed in a flask, and the content of the flask is stirred for 2 hr at a flask inside temperature of 60°C to give a reaction mixture. Then, the inside temperature of the flask is maintained at 25°C, sodium methoxide (11.4 g) is added to the flask while stirring the content of the flask, and the mixture is maintained as it is for 2 hr. Then, the inside temperature of the flask is maintained at 25°C, acetone (150 g) is added to the flask while stirring the content of the flask, and the mixture is reacted as it is at 25°C for 5 hr.

The content of the flask is analyzed. As a result, it is confirmed that compound (1¹) is produced in a yield of 99% based on cyanuric chloride. The content of the flask is filtered and the filtrate is recovered to give a reaction mixture containing compound (1¹).

### [Examples 10 - 14]

In the same manner as in Example 9 except that the use embodiment of sodium methoxide (itself or methanol solution), the amount of acetone used and the reaction temperature after adding acetone are changed, compound (1¹) was synthesized. The results are collectively shown in Table 2.

The "itself" in the Table means that solid sodium methoxide was directly added to the flask.

**[Table 2]**

| Example | NaOMe embodiment | acetone amount [eq] | reaction temperature [°C] | yield [%] |
|---|---|---|---|---|
| Example 9 | itself | 39 | 25 | 99 |
| Example 10 | itself | 9 | 40 | 69 |
| Example 11 | itself | 9 | 90 | 97 |
| Example 12 | itself | 6 | 90 | 92 |
| Example 13 | itself | 3.0 | 90 | 70 |
| Example 14 | methanol solution | 6 | 90 | 61 |

| | | | | |
|---|---|---|---|---|
| * Acetone amount is molar ratio based on cyanuric chloride. | | | | |

### [Example 15]

In a reactor under a nitrogen atmosphere were placed the compound (1¹) obtained in Example 9, triethylamine and methylene chloride to prepare a solution. Then, at room temperature, CHF₂C(O)F in a gas state is introduced into the reactor while stirring the content of the reactor and allowed to react. After the reaction, water is added to the reactor while ice-cooling the reactor, and the first organic phase containing the following compound (6¹) is recovered.

A 40% aqueous methylhydrazine solution and methylene chloride are added to the reactor under a nitrogen atmosphere to prepare a solution. Then, at -20°C, the first organic phase is introduced into the reactor while stirring the content of the reactor and allowed to react. After the reaction, water is added to the reactor and the second organic phase is recovered. The second organic phase is dried over sodium sulfate and evaporated under reduced pressure to give the following compound (8¹).

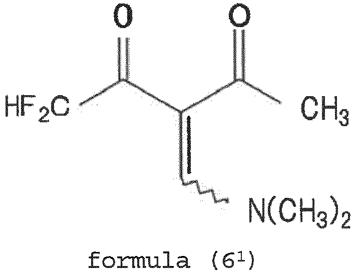

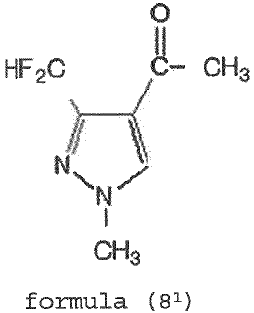

The present invention can provide a method for producing a nitrogen-containing compound useful as an optical material or a starting material for pharmaceuticals/agrochemicals industrially efficiently from an easily obtainable, economical compound.

## Claims

1. A method for producing a compound represented by the following formula (1), comprising reacting a compound represented by the following formula (4) with 8- to 40-fold moles of a compound represented by the following formula (3) to give a reaction mixture of the compound represented by the following formula (4) and the compound represented by the following formula (3), and reacting the reaction mixture with a compound represented by the following formula (2) by using a basic compound to give a compound represented by the following formula (1): 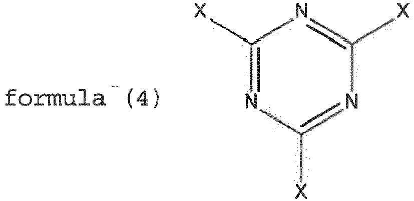
the
formula (3) NR¹R²C(O)H
the
formula (2) R³C(O)CH₃
the
formula (1) R³C(O)CH=CHNR¹R²
wherein X is a halogen atom, R¹ and R² are each independently an alkyl group having 1 - 6 carbon atoms, and R³ is a methyl group.

2. The production method according to claim 1, wherein the above-mentioned reaction mixture is mixed with a basic compound to give a mixture, the mixture is mixed with the above-mentioned compound represented by the formula (2), and the above-mentioned reaction mixture is reacted with the above-mentioned compound represented by the formula (2).

3. The production method according to claim 1 or 2, wherein the reaction mixture of the above-mentioned compound represented by the formula (4) and the above-mentioned compound represented by the formula (3) is obtained in the presence of ether.

4. The production method according to any one of claims 1 to 3, wherein the above-mentioned basic compound is alkali metal alkoxide.

5. The production method according to claim 4, wherein the above-mentioned alkali metal alkoxide is a solid alkoxide of an alkali metal.

6. The production method according to claim 4 or 5, wherein the above-mentioned compound represented by the formula (2) is used in an amount of more than 3-fold moles and not more than 20-fold moles relative to the above-mentioned compound represented by the formula (4), and the above-mentioned reaction mixture is reacted with the above-mentioned compound represented by the formula (2) by using a basic compound at a reaction temperature exceeding 40°C.

7. The production method according to claim 4 or 5, wherein the above-mentioned compound represented by the formula (2) is used in an amount of more than 12-fold moles relative to the above-mentioned compound represented by the formula (4), and the above-mentioned reaction mixture is reacted with the above-mentioned compound represented by the formula (2) by using a basic compound at a reaction temperature of not more than the boiling point of the above-mentioned compound represented by the formula (2).

8. A method for producing a compound represented by the above-mentioned formula (1), comprising obtaining a reaction product containing a compound represented by the above-mentioned formula (1) by the production method according to any one of claims 1 to 7, reducing or removing a low boiling point compound contained in the reaction product from the reaction product by an evaporation operation to give a crudely purified product, and distilling the crudely purified product to give a purified compound represented by the above-mentioned formula (1) .

9. The production method according to claim 8, wherein the low boiling point compound contained in the above-mentioned reaction product is reduced or removed from the above-mentioned reaction product by the evaporation operation at less than 70°C to give the crudely purified product.

10. A method for producing a compound represented by the following formula (8), comprising obtaining a compound represented by the above-mentioned formula (1) by the production method according to any one of claims 1 to 9, reacting the compound represented by the formula (1) with a compound represented by the following formula (5) to give a compound represented by the following formula (6), and reacting the compound represented by the formula (6) with a compound represented by the following formula (7):
the
formula (5) R^{F}C(O)Z
the
formula (7) R⁴NHNH₂ 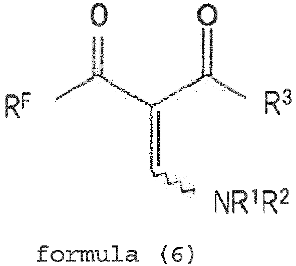 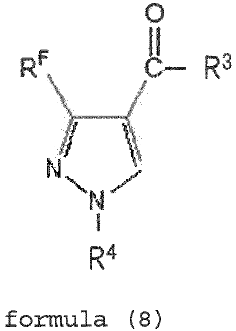
wherein Z is a fluorine atom or a chlorine atom, R^{F} is a haloalkyl group having 1 - 3 carbon atoms, R¹ and R² are each independently an alkyl group having 1 - 6 carbon atoms, R³ is a methyl group and R⁴ is an alkyl group having 1 - 3 carbon atoms.

## Patentansprüche

1. Verfahren zur Herstellung einer Verbindung der folgenden Formel (1), umfassend das Umsetzen einer Verbindung der folgenden Formel (4) mit dem 8- bis 40-fachen Mol einer Verbindung der folgenden Formel (3), um ein Reaktionsgemisch aus der Verbindung der folgenden Formel (4) und der Verbindung der folgenden Formel (3) zu ergeben, und das Umsetzen des Reaktionsgemisches mit einer Verbindung der folgenden Formel (2) unter Verwendung einer basischen Verbindung, um eine Verbindung der folgenden Formel (1) zu ergeben: 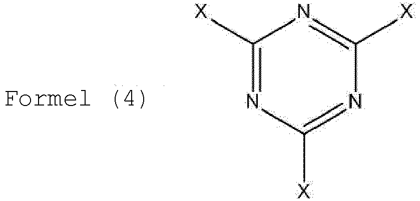
die
Formel (3) NR¹R²C(O)H
die
Formel (2) R³C(O)CH₃
die
Formel (1) R³C(O)CH=CHNR¹R²
wobei X ein Halogenatom ist, R¹ und R² jeweils unabhängig voneinander eine Alkylgruppe mit 1-6 Kohlenstoffatomen sind und R³ eine Methylgruppe ist.

2. Herstellungsverfahren nach Anspruch 1, wobei das oben genannte Reaktionsgemisch mit einer basischen Verbindung gemischt wird, um ein Gemisch zu ergeben, wobei das Gemisch mit der oben genannten Verbindung der Formel (2) gemischt wird und das oben genannte Reaktionsgemisch mit der oben genannten Verbindung der Formel (2) umgesetzt wird.

3. Herstellungsverfahren nach Anspruch 1 oder 2, wobei das Reaktionsgemisch der oben genannten Verbindung der Formel (4) und der oben genannten Verbindung der Formel (3) in Gegenwart von Ether erhalten wird.

4. Herstellungsverfahren nach einem der Ansprüche 1 bis 3, wobei die oben genannte basische Verbindung Alkalimetallalkoxid ist.

5. Herstellungsverfahren nach Anspruch 4, wobei das oben genannte Alkalimetallalkoxid ein festes Alkoxid eines Alkalimetalls ist.

6. Herstellungsverfahren nach Anspruch 4 oder 5, wobei die oben genannte Verbindung der Formel (2) in einer Menge von mehr als dem 3-fachen Mol und nicht mehr als dem 20-fachen Mol, bezogen auf die oben genannte Verbindung der Formel (4), verwendet wird und das oben genannte Reaktionsgemisch mit der oben genannten Verbindung der Formel (2) unter Verwendung einer basischen Verbindung bei einer Reaktionstemperatur über 40°C umgesetzt wird.

7. Herstellungsverfahren nach Anspruch 4 oder 5, wobei die oben genannte Verbindung der Formel (2) in einer Menge von mehr als dem 12-fachen Mol relativ zur oben genannten Verbindung der Formel (4) verwendet wird ) und das oben genannte Reaktionsgemisch mit der oben genannten Verbindung der Formel (2) unter Verwendung einer basischen Verbindung bei einer Reaktionstemperatur von nicht mehr als dem Siedepunkt der oben genannten Verbindung der Formel (2) umgesetzt wird.

8. Verfahren zur Herstellung einer Verbindung der oben genannten Formel (1), umfassend das Erhalten eines Reaktionsprodukts, das eine Verbindung der oben genannten Formel (1) enthält, durch das Herstellungsverfahren gemäß einem der Ansprüche 1 bis 7, das Reduzieren oder Entfernen einer im Reaktionsprodukt enthaltenen Verbindung mit niedrigem Siedepunkt aus dem Reaktionsprodukt durch einen Verdampfungsvorgang, um ein grob gereinigtes Produkt zu ergeben, und Destillieren des grob gereinigten Produkts, um eine gereinigte Verbindung der oben genannten Formel (1) zu ergeben.

9. Herstellungsverfahren nach Anspruch 8, wobei die im oben genannten Reaktionsprodukt enthaltene Verbindung mit niedrigem Siedepunkt durch den Verdampfungsvorgang bei weniger als 70°C reduziert oder aus dem oben genannten Reaktionsprodukt entfernt wird, um das grob gereinigte Produkt zu °ergeben .

10. Verfahren zur Herstellung einer Verbindung der folgenden Formel (8), umfassend das Erhalten einer Verbindung der oben genannten Formel (1) durch das Herstellungsverfahren gemäß einem der Ansprüche 1 bis 9, das Umsetzen der Verbindung der folgenden Formel (1) mit einer Verbindung der folgenden Formel (5), um eine Verbindung der folgenden Formel (6) zu ergeben, und Umsetzen der Verbindung der Formel (6) mit einer Verbindung der folgenden Formel (7):
die
Formel (5) R^{F}C(O)Z
die
Formel (7) R⁴NHNH₂ 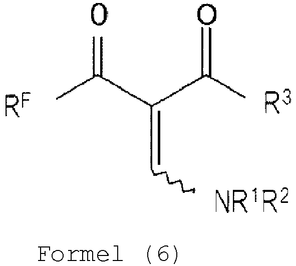 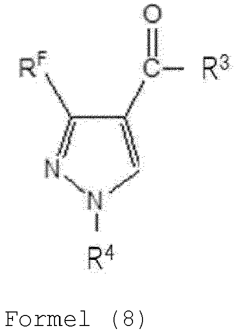
wobei Z ein Fluoratom oder ein Chloratom ist, R^{F} eine Halogenalkylgruppe mit 1-3 Kohlenstoffatomen ist, R¹ und R² jeweils unabhängig voneinander eine Alkylgruppe mit 1-6 Kohlenstoffatomen sind, R³ eine Methylgruppe ist und R⁴ eine Alkylgruppe mit 1-3 Kohlenstoffatomen ist.

## Revendications

1. Procédé pour la production d'un composé représenté par la formule (1) suivante, comprenant la réaction d'un composé représenté par la formule (4) suivante avec 8 à 40 fois en mole d'un composé représenté par la formule (3) suivante pour fournir un mélange réactionnel du composé représenté par la formule (4) suivante et du composé représenté par la formule (3) suivante, et la réaction du mélange réactionnel avec un composé représenté par la formule (2) suivante en utilisant un composé basique pour fournir un composé représenté par la formule (1) suivante : 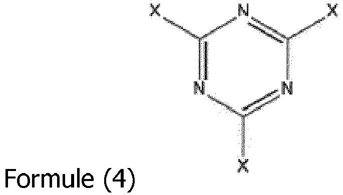
la
formule (3) NR¹R²C(O)H
la
formule (2) R³C(O)CH₃
la
formule (1) R³C(O)CH=CHNR¹R²
où X est un atome d'halogène, R¹ et R² sont chacun indépendamment un groupe alkyle ayant 1-6 atomes de carbone, et R³ est un groupe méthyle.

2. Procédé de production selon la revendication 1, où le mélange réactionnel cité ci-dessus est mélangé avec un composé basique pour fournir un mélange, le mélange est mélangé avec le composé cité ci-dessus représenté par la formule (2), et le mélange réactionnel cité ci-dessus réagit avec le composé cité ci-dessus représenté par la formule (2).

3. Procédé de production selon la revendication 1 ou 2, où le mélange réactionnel du composé cité ci-dessus représenté par la formule (4) et du composé cité ci-dessus représenté par la formule (3) est obtenu en présence d'éther.

4. Procédé de production selon l'une quelconque des revendications 1 à 3, où le composé basique cité ci-dessus est un alcoxyde de métal alcalin.

5. Procédé de production selon la revendication 4, où l'alcoxyde de métal alcalin cité ci-dessus est un alcoxyde solide d'un métal alcalin.

6. Procédé de production selon la revendication 4 ou 5, où le composé ci-dessus représenté par la formule (2) est utilisé dans une quantité supérieure à 3 fois en mole et d'au plus 20 fois en mole par rapport au composé cité ci-dessus représenté par la formule (4), et le mélange réactionnel cité ci-dessus réagit avec le composé cité ci-dessus représenté par la formule (2) en utilisant un composé basique à une température de réaction excédant 40°C.

7. Procédé de production selon la revendication 4 ou 5, où le composé cité ci-dessus représenté par la formule (2) est utilisé dans une quantité supérieure à 12 fois en mole par rapport au composé cité ci-dessus représenté par la formule (4), et le mélange réactionnel cité ci-dessus réagit avec le composé cité ci-dessus représenté par la formule (2) en utilisant un composé basique à une température de réaction d'au plus le point d'ébullition du composé cité ci-dessus représenté par la formule (2).

8. Procédé de production d'un composé représenté par la formule (1) citée ci-dessus, comprenant l'obtention d'un produit de réaction contenant un composé représenté par la formule (1) cité ci-dessus par le procédé de production selon l'une quelconque des revendications 1 à 7, la réduction ou l'élimination d'un composé de faible point d'ébullition contenu dans le produit de réaction à partir du produit de réaction par une opération d'évaporation pour fournir un produit grossièrement purifié, et la distillation du produit grossièrement purifié pour fournir un composé purifié représenté par la formule (1) citée ci-dessus.

9. Procédé de production selon la revendication 8, où le composé de faible point d'ébullition contenu dans le produit de réaction cité ci-dessus est réduit ou éliminé du produit de réaction cité ci-dessus par l'opération d'évaporation à moins de 70°C pour fournir le produit grossièrement purifié.

10. Procédé de production d'un composé représenté par la formule (8) suivante, comprenant l'obtention d'un composé représenté par la formule (1) citée ci-dessus par le procédé de production selon l'une quelconque des revendications 1 à 9, la réaction du composé représenté par la formule (1) avec un composé représenté par la formule (5) suivante pour fournir un composé représenté par la formule (6) suivante, et la réaction du composé représenté par la formule (6) avec un composé représenté par la formule (7) suivante :
la
formule (5) R^{F}C(O)Z
la
formule (7) R⁴NHNH₂ 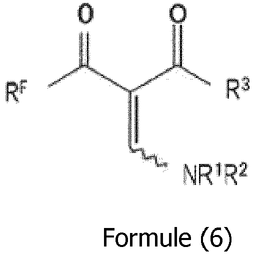 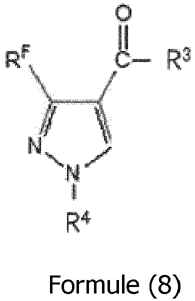
où Z est un atome de fluor ou un atome de chlore, R^{F} est un groupe haloalkyle ayant 1-3 atomes de carbone, R¹ et R² sont chacun indépendamment un groupe alkyle ayant 1-6 atomes de carbone, R³ est un groupe méthyle et R⁴ est un groupe alkyle ayant 1-3 atomes de carbone.
